# EUROPEAN PATENT SPECIFICATION

(11) **EP 1 900 345 B1**
(45) Date of publication and mention of the grant of the patent: **03.04.2013**
(21) Application number: 05774476.5
(22) Date of filing: 06.07.2005
(51) Int. Cl.: A61F 2/38

(54) **WHOLE KNEE PROSTHESIS**
KNIETOTALPROTHESE
PROTHESE TOTALE DU GENOU

(43) Date of publication of application: 19.03.2008
(73) Proprietor: Perez Cosias, German, 46980 Paterna (ES)
(72) Inventor: Perez Cosias, German, 46980 Paterna (ES)
(74) Representative: Tari Lazaro, Aida
(86) International application number: PCT/ES2005/070099
(87) International publication number: WO 2007/023196

(56) References cited:
- EP-A- 0 985 386
- EP-A- 1 004 283
- US-A- 6 086 614
- US-A- 6 126 692
- US-A- 6 146 424
- US-A1- 2003 204 268

## Description

### OBJECT OF THE INVENTION

The present invention relates to complete knee prosthesis comprising a revision femoral component, a patellar insert, a tibial insert plus a metallic tibial tray along with means of attaching the inserts to the femur and tibia respectively.

The femoral component presents a cylinder that has been machined internally in order to permit its attachment to the femur by means of a rod. A perfect adaptation of the position of the rod to the femur is achieved since the rod can be straight or oblique in order to be adapted to the anatomy of each patient.

Moreover, once it has been prefixed, said rod is guided by the medullary canal without invading undesired zones, which would imply consequent damage to the bone, until the rod occupies its final position and is fastened once and for all by the surgeon.

The tibial tray is also characterised in that it presents a cylinder machined internally to which two rods can likewise be adapted, one inclined and the other straight, and in that it presents a keel which provides increased stability and resistance to rotation with minimum impact on the bone. Five models of metallic tibial tray are provided.

These prosthesis can also be carried out with three different models of tibial inserts where these three models are fully interchangeable with the five metallic tray models.

This new knee prosthesis succeeds in providing the surgeon with greater flexibility during the surgical operation, due to being adapted better and more easily to the anatomy of each patient. The number of pieces necessary in order to obtain a greater number of sets of knee prosthesis is reduced, thereby lowering the manufacturing costs.

### PRIOR ART OF THE INVENTION

Prostheses are known for the knee joint which consist of a femoral component, tibial insert, rotating insert and means of securing and adjustment.

The customary practice is for the bone fastenings for the inserts to be made by means of screwing into the bone with pins.

On other occasions the femoral and tibial components include rods that are independent of the inserts which are seated inside the medullary canal providing increased stability on account of their greater length.

There exist knee prostheses which also permit an angular adjustment to be made of the femoral and tibial components to the human anatomical curvature. These angular adjustments are usually performed with the coupling of different independent pieces such as a rod and rod collar joined with a connector and fastened to each other and to the insert by means of a pin.

The adjustment angles that are achieved usually vary between 0° and 9°, which are the most customary in human anatomy. The way of achieving this can be either because the pieces are made with different angles or because of the form of carrying out the seating of the pin in the insert. As reference to these fastening systems for the femoral component, one can cite patents with publication numbers EP0714645 and EP0376658.

Moreover, patents with publication numbers EP0853930, FE2748389 and US55290313 present a knee prosthesis with two degrees of freedom for better coupling to the medullary canal.

Nevertheless, the above prostheses have to have their final position defined prior to being inserted in the medullary canal of the patient, which can lead to intrusion into undesired areas of the bone.

A knee prothesis as defined in the preamble of claim 1 is disclosed in US-A-6 086 614.

The present invention seeks to facilitate the surgeon in the surgical operation of the complete knee prosthesis implant, furthermore permitting the implants to be fully adapted to the anatomy of each patient due to the fact that the rod is guided by the medullary canal without invading undesired zones, which would imply consequent damage to the bone, until the rod occupies the final position and is fastened once and for all by the surgeon.

To do this, the surgeon will be able to choose between two rods for each one of the inserts, a straight rod and another oblique rod, which are adjusted directly in some support cylinders machined internally and which project from the femoral component and from the tibial insert. By simply rotating the rods in the cylinders, the surgeon achieves the required angle of insertion guided by the medullary canal, without the need for any other additional piece with the exception of the pins which provide the final fastening of the rods with the inserts.

With this new complete knee prosthesis, the number of pieces needed for replacing the joint is efficiently reduced and thereby becomes more economical.

There also exists a complete interchangeability between the tibial inserts and the tibial trays, which allows a surgeon to choose among a greater number of sets when it comes to performing the surgical operation.

### DESCRIPTION OF THE INVENTION

The present invention relates to a complete knee prosthesis. It consists of a prosthetic unit comprising a revision femoral component, a tibial component, a rotating component and a set of rods and pins.

The knee is the largest joint in the human body. It is responsible for the transmission of loads in the lower limb and participates in its movement. The knee has to possess great stability since when extension is complete it has to support the entire weight of the body on a relatively small surface. At the same time, it has to be endowed with great mobility in order to be able to run or walk.

In the event of serious injury in which the knee needs to be replaced with artificial mechanical elements, the prosthesis has the main objective of recovering the functionality of the knee. The invention that is described here achieves these objectives by means of the components detailed below.

This novel complete knee prosthesis is intended to replace the knee joint in cases in which the cruciate ligaments are also unusable.

According to one embodiment the components for this consist of:
- a revision femoral component which has two condylar surfaces joined by a connector in the form of a cavity from which projects an inclined support cylinder machined internally for its attachment to the femur by means of a rod,
- a tibial insert that gives support to the condylar surfaces of the femoral component and which fits by clipping onto a tibial tray,
- a tibial tray in the form of a plate which in its lower face has a keel and a cylinder with its axis perpendicular to the plate which is also machined internally for adjustment to the tibia with a rod,
- straight and oblique rods machined for their adjustment in the cylinders of the femoral component and of the tibial insert and which attach them to the femur and tibia respectively,
- fastening pins for the rods to the inserts,
- a rotating component.

The femoral component of the prosthesis reproduces the lower part of the femur. In this way, two surfaces are observed which simulate the condyles of the femur and which are connected by an intermediate cavity. Emerging from the upper part of this cavity is a cylinder inclined in the medio-lateral plane which permits coupling of a rod, straight or oblique, for its attachment to the femur.

The cylinder is machined internally with a through-hole. The diameter of the internal machining of the cylinder is less from approximately half of the cylinder as far as the cavity for housing the head of the fastening pin.

One of the objectives of the invention essentially consists of means of coupling between the rod and the support cylinder, having the capacity for adjustment of the inclination of the rod with respect to its base piece. This objective is achieved thanks to a particular way of carrying out the join between the rod and the support cylinder as described below.

Two types of rod can be fitted to the support cylinder mentioned above: straight or oblique. Both rods have a pivot with a truncated conical shape at one of their ends which will be the one that is introduced into the upper part of the cylinder.

The oblique rod has the axis of the truncated conical pivot inclined with respect to the axis of the rod. For most of the cases considered it has been confirmed that by taking an angle of 5° the capacity of the prosthesis to adapt to the patient's anatomy is sufficient. The fact of considering this angle does not mean that the invention cannot be carried out with different angles. The inclination of the cylinder with respect to the vertical together with the inclination of the axis of the rod permits variations in the angle to be achieved of between 0° and the sum of the inclinations of the axes of the cylinder and of the rod simply by turning the oblique rod in the support cylinder. This angular variation will typically lie between 0° and 10° since one usually considers oblique rods with an inclination of the axis of 5° and an inclination also of 5° for the support cylinder.

The angular variation permitted by the oblique rod and which make it possible to use a straight rod for the same femoral component allows the surgeon to adapt the insert in the most suitable way according to the morphology of the femur of each patient.

As well as what has been stated above, another of the main objectives of the invention is the fact that the rod, by means of a pre-tightening to the cylinder, is guided by the medullary canal without invading undesired zones, which would imply consequent damage to the bone, until the rod occupies the final position and is fastened once and for all by the surgeon.

The upper part of the tibia is reproduced by a tibial component comprising a tibial insert intended to establish the support for the condyles of the femoral component and a tray, preferably made of polyethylene, to which the tibia is attached by means of a rod.

Different types of tibial inserts which correspond to their femoral pieces can fit in a single tibial tray. The way of fitting both pieces will be described below.

The tibial tray has a cylinder which arises perpendicular to the lower central part of the tray. This cylinder has the same purpose as stated previously for the femoral component.

This cylinder allows two different rods, straight and oblique, to be adjusted with it, in the same way as has been described for the femoral component though in this case they have a shorter length. It will be for the surgeon to decide which one is better adapted to the anatomy of each patient.

The manner of adjusting the cylinder with the rods is also done in the same way as has been indicated with the femoral component.

The cylinder also provides a housing for the head of the fastening pin which provides the temporary and permanent fastening between the tibial tray and the rod.

The tray also has a keel which consists of two flaps emerging radially from the cylinder and which form an angle of 120° between them, and which have their widest part in the outermost zone. This arrangement provides protection for the knee against rotations.

The manner of adjusting the different models of tray permits complete interchangeability with any of the tibial inserts mentioned, no matter what their size.

The manner of fitting the tibial tray and tibial insert will be by clipping. The tray has a projection in the form of a guide. This projection permits a hinged support between the two pieces. The final adjustment by clipping takes place by means of inserting a clip. In any of the models, in order to permit interchangeability between the tibial trays and inserts, the distance between the guide and the dovetail shape section is kept constant.

Each of the different tibial inserts is produced in different sizes and they are all interchangeable with any of the tibial trays, which leads to a reduction in the number of pieces needed in order to obtain a large number of different sets of prosthesis, thereby lowering the manufacturing costs.

The tibial component and the femoral component of the prosthesis that is implanted in the right knee has a symmetric geometry with respect to the tibial component and the femoral component of the prosthesis that is implanted in the left knee.

### DESCRIPTION OF THE DRAWINGS

This descriptive specification is complemented with a set of plans, illustrating the preferred, though at no time limiting, example of the invention.
Figure 1 shows the revision femoral component in which for clarity of the drawing just the most characteristic parts of this piece are shown in a broken line.
Figure 2 shows the revision femoral component and a coupled rod.
Figure 3 shows in the upper left-hand part a front view of the attachment system of the tibial tray, in the lower left-hand part an exploded view of a model of tibial insert together with a tibial tray and a clip, and on the right a section and detail of the tibial insert, the tibial tray and the clip already fitted.
Figure 4 shows a front view in plan of a tray coupled to a tibial tray and oblique rod.
Figure 5 is a representation of the two rods, straight and oblique, of the femoral component.

### DETAILED EXPLANATION OF VARIOUS FORMS OF EMBODIMENT

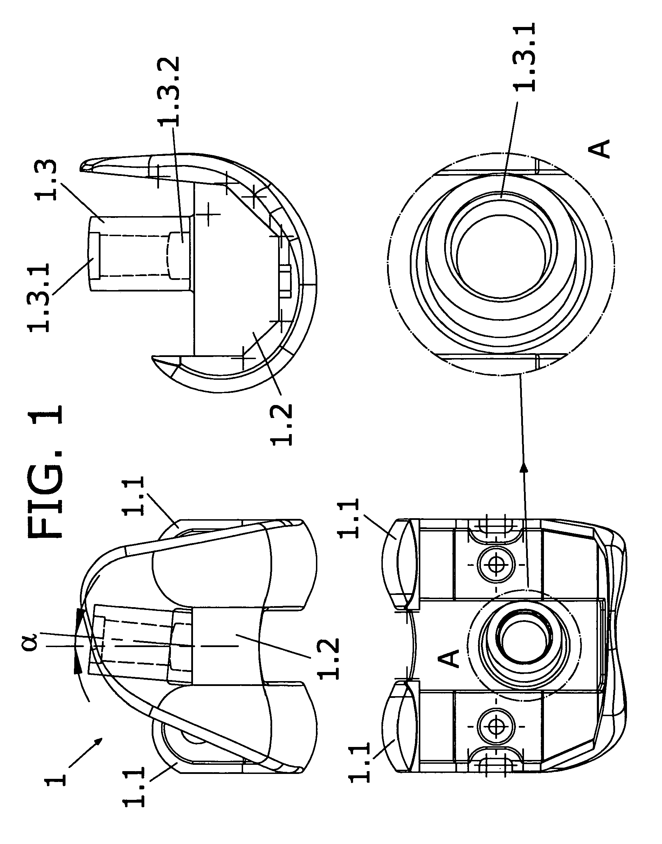

Figure 1 is a representation of the revision femoral component (1). In it can be seen the condylar surfaces (1.1) which reproduce the condyles of the femur. These condylar surfaces (1.1) are connected by an intermediate cavity (1.2) projecting from the top of which is a support cylinder (1.3) which is inclined by 5° in a medio-lateral plane.

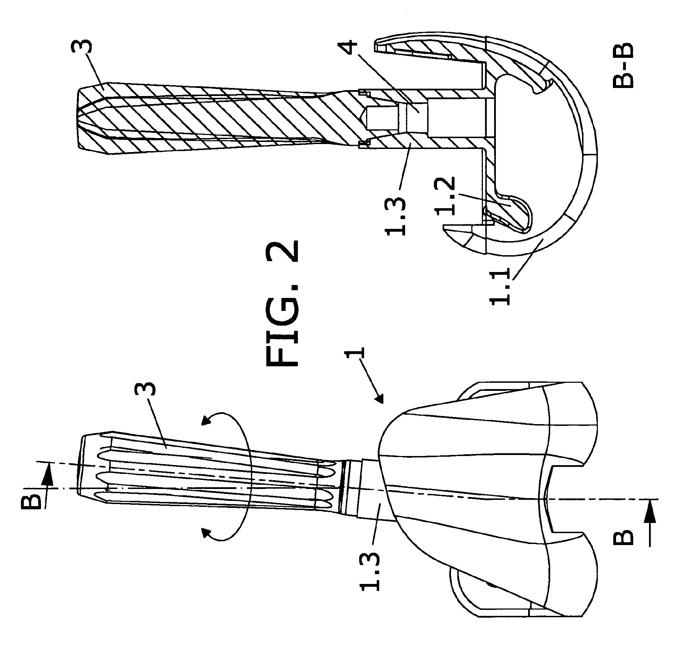

The support cylinder (1.3) is intended to provide support for a straight rod (2) or oblique rod (3), as can be seen in Figure 2.

In order for the surgeon to achieve the desired adjustment at the moment of the operation according to the anatomy of each patient, the support cylinder (1.3) is inclined by an angle α which in this embodiment is 5°, where either a straight rod (2) or an oblique rod (3) can be coupled indifferently, with a pre-tightening being carried out by means of a pin (4). Following this coupling the surgeon can achieve the angular variation required by the patient's anatomy since it is the medullary canal which guides the rod (2, 3) owing to the existence of the pre-tightening, with which at no time are any undesired zones invaded.

The angular variations range between 0° and 10° simply by causing the oblique rod (3) to rotate in the support cylinder (1.3) since in this example the angle of inclination β of the oblique rod (3) is 5°. In the event that the 5° inclination of the support cylinder (1.3) is an inclination that is a priori suited to the patient's anatomy, then the straight rod (2) can be replaced with the oblique rod (3), with which it will be easier to position the rod (3) inside the medullary canal.

Also to be seen in Figure 1 is a detail of a circular recess (1.3.1) located in the supper part of the support cylinder (1.3) and in its interior diameter. The straight or oblique rods (2, 3) rest on this circular recess (1.3.1).

The final fastening of either of the rods (2, 3) to the revision femoral component (1) is done by means of the final tightening of the pin (4). The housing (1.3.2) for the head of the pin can also be seen in Figure 1. This housing (1.3.2) is an internal machining in the lower part of the support cylinder (1.3).

Figure 2 shows a front view in section of the assembly of an oblique rod (3) with the revision femoral component (1).

It can be seen from the front view how, by rotating the oblique rod (3) in the support cylinder (1.3), angular variations are achieved from between 0° and 10° with respect to the vertical. These limits are determined by the 5° of the support +/- the 5° of the base of the oblique pin.

The section B-B allows one to see how the final adjustment of the rod (3) and the femoral component (1) is made by means of the rod (4).

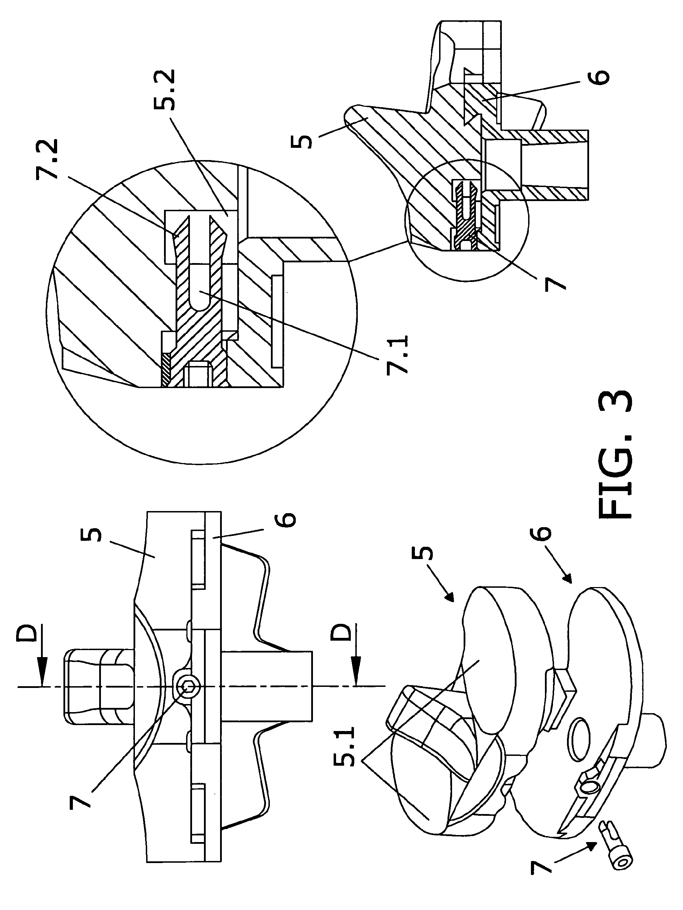

The bottom left-hand part of Figure 3 shows a model of tibial insert (5), typically manufactured in plastic, preferably polyethylene, together with a metallic tibial tray (6) and a clip (9) where the projections of the metallic tibial tray (6) coincide with the gaps in the tibial insert (5), permitting a hinged support. The condylar housings (5.1) can be seen, which provide support for the condylar surfaces (1.1) of the femoral component (1).

In the right-hand part of the Figure 3 can be seen the section D-D in which a detail has been blown up of the attachment system by clipping of the tibial insert (5) and the metallic tibial tray (6), where the clip (9) presents a transverse slot (9.1) which permits deformation of its end (9.2) in the form of an arrow head until it becomes housed in a hole (5.2) in the tibial insert (5).

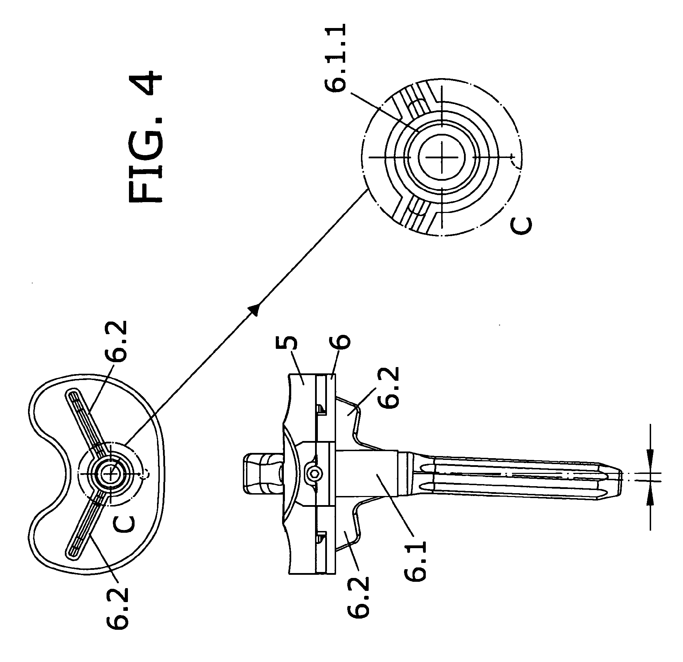

Figure 4 is a front view of the tibial insert (5) fitted on a metallic tibial tray (6). Projecting perpendicularly from the lower part of the metallic tibial tray (6) is a cylinder (6.1) in order to provide a housing for some rods (2, 3) of shorter length than in the case of the femoral component (1). These rods can be straight (2) or oblique (3).

The manner of adjusting the rods (2, 3) to the metallic tibial tray (6) is via a circular recess (6.1.1) in the cylinder in the same way as described for the femoral component (1).

In the front view and plan view of Figure 4 it can be seen that the metallic tibial tray (6) is also provided with a keel (6.2) formed from two flaps which arise radially from the cylinder (6.1) and which between them form an angle of around 120°. Detail C shows the circular recess (6.1.1) made in the cylinder (6.1).

The flaps forming the keel (6.2) are intended to provide protection for the knee against rotations, hence the fact that the outer section is wider, as can be seen in Figure 4. With this, the moment of inertia of the piece manages to be increased, at the same time maintaining a small section and thereby minimising the impact on the bone.

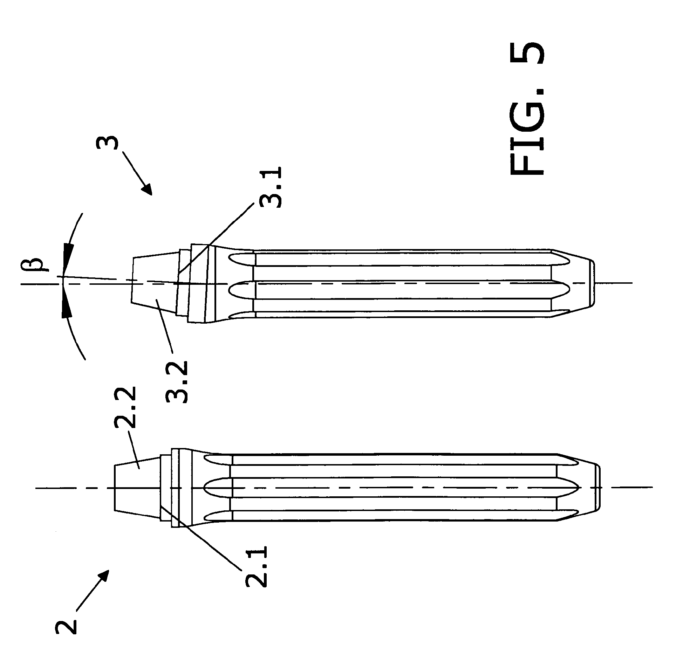

Figure 5 shows the rods (2, 3) for attachment to the bone of the femoral component (1). There is a straight rod (2) and an oblique rod (3).

In the rods (2, 3) a truncated conical pivot (2.2, 3.2) can be seen which is the one that is introduced into the support cylinder (1.3) of the femoral component (1) where a seating (2.1, 3.1) acts as the support in the circular recess (1.3.1).

The oblique rod (3) has the axis (3.4) of the truncated conical pivot (3.2) inclined at an angle β which in this embodiment is 5° with respect to the axis of the rod (3.3). This inclination is what permits angular variations to be obtained of from 0° to 10° simply by rotating the rod in the support cylinder (1.3) of the femoral component (1), which also has its axis inclined at an angle α of 5° with respect to the vertical.

The rods (2, 3) that are coupled in the cylinder (6.1) of the tibial insert (6) are like those just described but of shorter length. Either a straight rod (2) or an oblique rod (3) can also be coupled indifferently in the cylinder of the tibial insert (6).

The femoral component (6) and tibial component described in this preferred embodiment correspond to a prosthesis for the right knee. This can be seen above all in the front view of Figure 1 on account of the inclination of the front projection towards the right which corresponds to the interior of the right knee. Said components corresponding to the right knee have a symmetrical geometry with the homologous components for the left knee.

The essential nature of this invention is not altered by variations in materials, form, size and arrangement of the component elements, described in a non-limiting manner, which suffices for an expert to proceed to its reproduction.

## Claims

1. Complete knee prosthesis that includes a femoral component (1), a tibial component, a rod, a patellar insert and means of fastening and adjustment the femoral and tibial components to the bone, said fastening and adjustment means comprising at least, one support cylinder (1.3; 6.1) joined to one of the two aforementioned components, having a truncated conical housing prepared to receive a truncated conical pivot (3.2) that extends from the rod (3) that is adapted to be inserted in the medullary channel of the bone, **characterized in that** the truncated conical pivot (3.2) and the rod (3) axis have an oblique configuration; in such a manner that when the rod (3) rotates on its truncated conical pivot (3.2) inside the truncated conical housing of the support cylinder, the rod's inclination changes orientation.

2. Complete knee prosthesis according to claim 1, **characterized in that** the tibial component comprises one tibial insert (5) and one tibial tray (6) joined by a clip (7) having a transversal slot (7.1) that allows its arrow end (7.2) to deform until it is inserted in a housing hole (5.2) of the tibial insert (5).

## Patentansprüche

1. Komplette Knieprothese, die eine Oberschenkelkomponente (1), eine Schienbeinkomponente, einen Schaft, einen Kniescheibeneinsatz sowie ein Hilfsmittel zur Befestigung und Anpassung der Oberschenkel- und Schienbeinkomponenten an den Knochen aufweist, **gekennzeichnet dadurch, dass** das genannte Hilfsmittel zur Befestigung und Anpassung mindestens einen Stützzylinder 1.3; 6.1 aufweist, der mit einer der zwei vorgenannten Komponenten verbunden wird und eine verkürzte Ummantelung für die Aufnahme eines verkürzten konischen Zapfens (3.2) hat, die vom Schaft (3) ausgeht, der für eine Einführung in den Markkanal des Knochens angepasst wird, **gekennzeichnet dadurch, dass** die Achse des verkürzten konischen Zapfens (3.2) und des Schafts (3) schräg konfiguriert sind; in einer Weise, dass bei Rotieren des Schafts (3) auf seinem verkürzten konischen Zapfens (3.2) innerhalb der verkürzten konischen Ummantelung des Stützzylinders ein Richtungswechsel bei der Neigung des Schafts eintritt.

2. Komplette Knieprothese nach Anspruch 1, **gekennzeichnet dadurch, dass** die Schienbeinkomponente einen Schienbeineinsatz (5) und eine Schienbeinplatte (6) aufweist, durch einen Clip (7) mit seitlicher Nut (7.1) befestigt, der seinem Pfeilende (7.2) eine Verformung bis zur Einführung in ein Loch der Ummantelung (5.2) der Schienbeineinsatz (5) erlaubt.

## Revendications

1. Prothèse totale du genou comprenant un élément fémoral (1), un élément tibial, une tige, un insert rotulien et des moyens d'attache et d'ajustement des éléments fémoraux et tibiaux à l'os, **caractérisés en ce que** ces moyens d'attache et d'ajustement comprennent, au minimum, un support cylindrique 1.3 ; 6.1 uni à l'un des deux éléments mentionnés précédemment, avec un boîtier conique tronqué préparé pour recevoir un pivot conique tronqué (3.2) qui s'étend depuis la tige (3) adaptée pour entrer dans la cavité médullaire de l'os, **caractérisé en ce que** l'axe du pivot conique tronqué (3.2) et de la tige (3) ont une configuration oblique ; de telle sorte que lorsque la tige (3) tourne sur son pivot conique tronqué (3.2) à l'intérieur du boîtier conique tronqué du support du cylindre, l'inclinaison de la tige change l'orientation.

2. Prothèse totale du genou selon la revendication 1, **caractérisée en ce que** la prothèse tibiale comprend un insert tibial (5) et un plateau tibial (6) reliés par une bague de serrage (7) présentant une fente transversale (7.1) qui permet à son extrémité en flèche (7.2) de se déformer jusqu'à entrer dans un trou de boîtier (5.2) de l'insert tibial (5).
